# EUROPEAN PATENT APPLICATION

(11) **EP 4 722 276 A1**
(43) Date of publication of application: **08.04.2026**
(21) Application number: 24203788.5
(22) Date of filing: 01.10.2024
(51) Int. Cl.: C08J 3/22

(54) **LOW MELTING CARBOXAMIDE HALS FOR IMPROVED STABILIZATION OF POLYAMIDES**

(71) Applicant: CLARIANT INTERNATIONAL LTD, 4132 Muttenz (CH)
(72) Inventor: SCHEIBELEIN, Christoph, 86152 Augsburg (DE); STEFFANUT, Pascal, 68730 Michelbach le bas (FR); AMNUAYPORNSRI, Sureerut, 10400 Bangkok (TH); SCHALLER, Thomas, 86391 Stadtbergen (DE); HIRSCHBECK, Jana, 86647 Buttenwiesen (DE)
(74) Representative: Zusammenschluss Clariant

(57) **Abstract**

The invention relates to a low molecular weight HALS stabilizer (LMW-HALS) for enhancing the processing and long-term stability and the heat resistance, light resistance and chemical resistance of polymer or resin. The present invention also relates to a stabilizer masterbatch and a composition comprising such stabilizer and a polymer or resin.

## Description

### Technical field

The present invention relates to the use of a low molecular weight HALS stabilizer (LMW-HALS) for enhancing the processing and long-term stability, such as the heat resistance, light resistance and oxidation resistance of polyamides.

The present invention also relates to a stabilizer/polyamide masterbatch and polyamide composition comprising such stabilizer.

### Background of the invention

Polyamides are widely used in industry to produce fibers or molded bodies by spinning or injection molding.

The service-life of polyamides is dependent on (1) raw materials used in the manufacturing process and the manufacturing process itself, (2) the melt processing and the additives used for stabilizing the polymer (stabilizers) against damage brought about by melt-processing and (3) environmental weathering factors.

Melt-processing is applied in extrusion processes, such as master batch preparation,spinning processes and injection molding procedures.

Synthetic polyamides are typically melt-processed at temperatures between 240°C and 300°C, and especially PA 6 is processed at only 240°C, which is roughly 20°C above its melting point. Heat stability issues can arise in the temperature range between 150°C and 300°C in that polyamides are decomposed by heat and oxygen, which cause the decomposition of the polymeric chains and therefore impact color changes and the loss of favorable mechanical properties. Therefore, heat stabilization against thermo-oxidative degradation processes is required.

Environmental weathering factors arise during the use of molded and extruded goods at ambient temperatures under heat, light and oxygen exposure. These negative long-term influences are not as intense as those caused by melt-processing, but over a longer period of time, they cause significant ageing and discoloration of the material and loss of the mechanical properties. Under these conditions polyamides require long-term stabilization against exposure to heat, light and oxygen.

To reduce these unfavored degradation effects, hindered amine light stabilizers (HALS) need to be added to the polyamide composition.

Hindered amine light stabilizers (HALS stabilizers), which act as self-regenerating radical scavengers are used in polyamides to reduce their degradation during melt-processing and to enhance long-term light stability at ambient temperatures. Especially sterically hindered cyclic amines are widely used in industry as polymer stabilizers.

In most cases they are a derivative of 2,2,6,6-tetramethylpiperidine.

They are generally produced using 2,2,6,6-tetra-methyl-4-piperidinyl (TAA) or from its derivatives, such as 2,2,6,6-tetramethylpiperidin-4-ol (TAAol) and 2,2,6,6-tetramethylpiperidin-4-amine (TAD), N-butyl-2,2,6,6-tetramethyl-piperidin-4-amine (Bu-TAD) or N,N'-bis(2,2,6,6-tetramethyl-4-piperidyl)hexane-1,6-diamine (HMBTAD). HALS compounds scavenge efficiently free radicals formed in plastics articles and in coatings or lacquers, especially at the surface, where minor or no protection through the UV absorber is given. This process has been extensively studied and is essentially a cyclic chain breaking antioxidant process which is known as the Denisov cycle.

WO9743335 discloses a well-known stabilizer for PA 6, namely N,N'-bis(2,2,6,6-tetramethyl-4-piperidinyl)-1 ,3-benzenedicarboxamide, which is also known as the commercial product Nylostab^{®} S-EED.

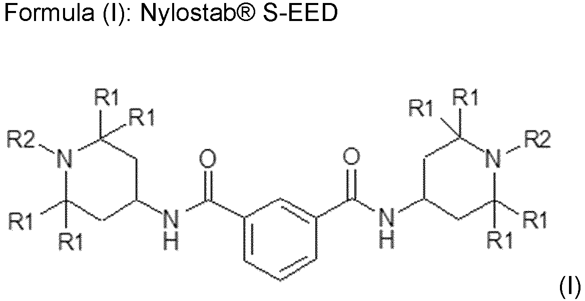

Nylostab^{®} S-EED is performing as multipurpose additive and allows process stability as well as light, heat and oxidation resistance.

WO9743335 describes, among other things, the use of a compound according to formula (I) which was produced with an alternative process for the stabilization of polyamides.

Due to its chemical structure, Nylostab^{®} S-EED is highly suitable for polyamides. It is usually introduced upstream during the polymerization process of polyamides due to its reasonably good solubility in ε-caprolactam and is a well-established process and light stabilizer for PA 6 as disclosed in EP0903372.

Alternatively, it can be added in downstream processes, such as compounding, extrusion, spinning, injection molding or masterbatch preparation.

However, the high melting point of about 274°C limits its use in compounding and extrusion to high melting polymers, as processing temperatures in extrusion must be adapted to at least 280°C to ensure dispersibility in the downstream processes.

Further, increased temperatures are unfavorable due to increased energy consumption and increased oxidation processes causing discoloration and loss of mechanical properties.

As known from industry it is always preferable to provide stabilizers which enhance the melt-processing stability and improve the light stability and color rentention of the polyamide.

Therefore, the problem addressed by the present invention is to increase melt-processing stability of polyamides, especially PA 6. Further the service-life of polyamides, especially PA 6, shall be extended by improving the light stability of the polyamide.

### Subject Matter of the invention

This problem has been solved by the use of a compound of formula (II) in polyamides:

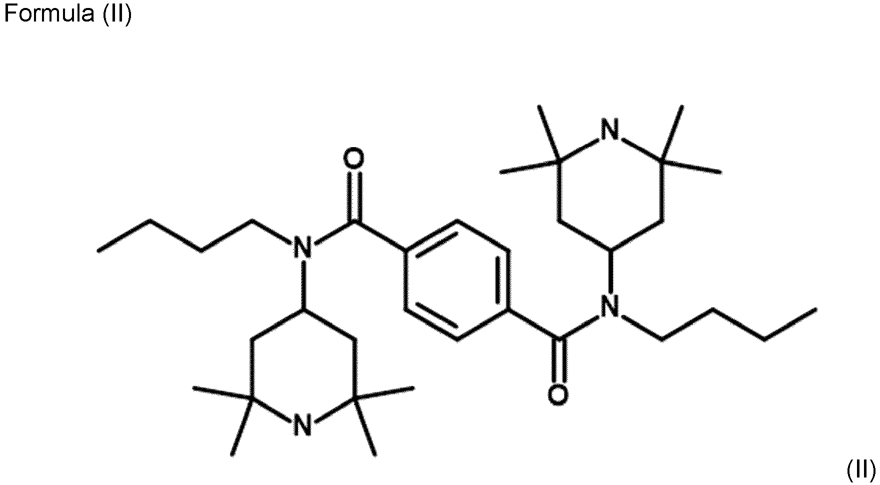

It was found that by incorporating the compound of formula (II) in a polyamide the light stability could be enhanced.

The compound of formula (II) is synthesized by condensation of a terephthalic acid chloride or it's dimethyl ester with a compound of formula (III):

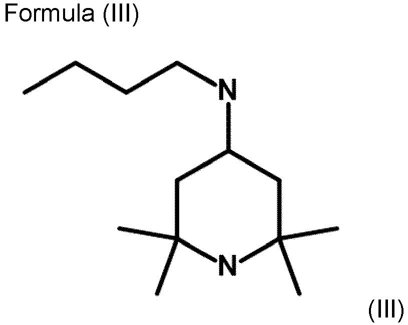

The compound of formula (II) is obtainable by condensation of terephthalic acid chloride or terephtalic dialkyl ester, preferably terephthalic acid chloride (TPC) or dimethyl terephthalate ester, most preferably terephthalic acid chloride (TPC) with two equivalents of the compound of formula (III), following processes described in the published applications WO2004016591 or WO2017/005413.

The compound of formula (III) is commercially available and is obtainable by a reductive amination of triacetone amine (TAA) using n-butylamine. This process is well-known in industry.

The compound of formula (II) is used for polyamides to improve its light stability.

Accordingly, a composition comprising polyamide and compound of formula (II) is also an object of the invention, as is a process for its preparation.

The compound of formula (II) can also be provided in the form of a masterbatch of a polyamide, so that the invention also relates to a polyamide masterbatch comprising a compound of formula (II).

The invention accordingly also provides a method for stabilization of polyamides, , which comprises admixing (a) the polyamide before or during processing or (b) the monomers used for the polymerization of the polyamide with an effective amount of the compound of formula (II).

The compound of formula (II) is added to a polyamide, preferably PA 6 or PA 66 in an amount of 0.01 to 10.0 wt%, preferably of from 0.05 to 5.0 wt%, more preferably of from 0.08 to 2.5 wt%, in particular of from 0.1 to 1.0 wt%, based on the total weight (100 wt%) of the total polymer composition. Thus, the polyamide composition shows a better color retention in melt-processing. Further, it shows a lower yellowing and/or discoloration tendency and has better mechanical properties after exposure to environmental weathering factors simulated by weatherometer stability tests.

The compound of formula (II) is particularly useful in polyamides, due to the good solubility in the monomers and the polyamides.

Polyamides stabilized according to the invention contain amide linkages (-CONH-) in the polymer backbone, which are synthesized from diamines, dicarboxylic acids, lactams, or amino acids. These polymers could be classified as aliphatic, semi-aromatic and aromatic polyamides. Examples include nylon 6 (PA 6), nylon- 6.6 (PA 66), nylon-6.10, nylon 6.11, nylon-6.12, nylon 11, nylon 12, and copolymers such as nylon-6.6/6, nylon-6.6/6.10, 6/11, 6/12; polyether-polyamide block copolymers, poly(m-phenylene-isophthalamide), poly(p-phenylene terephthalamide), and the like all of which are commercially available from a variety of sources.

The methods for synthesizing polyamide polymers, comprise step-growth polymerization, ring-opening polymerization, anionic polymerization, cationic polymerization, or interfacial polymerization.

The method includes using the stabilization system with formulated polyamides, including conventional impact toughened polyamide, and reinforced polyamides containing glass fiber, mineral and glass/mineral combinations. The invention can be practiced with polyamides regardless of the polymer morphology. Amorphous, semicrystalline or highly crystalline polyamides as well as blends of different crystallinity are benefited. Commercially available polyamide resins are known from sources such as BASF, DSM, Evansville, Ind. and E. I. duPont de Nemours; formulated polyamides are available, for example, from Ferro Corp., A. Schulman and Akron; reinforced polyamides are available from DSM and Westlake.

Polyamides, used with compound from formula (II) of the invention are useful in melt-processing operations as sheet and fiber extrusion and co-extrusion as well as blow molding, injection molding and rotary molding.

Fibers include melt spinning, solution spinning and melt blown fiber operations for use in woven or non-woven form to make filters, diaper fabrics, medical garments, geotextiles, etc.

Molded articles include single and multi-layered constructions in the form of tanks, large hollow articles and automotive applications, etc.

The present invention further provides a polyamide composition comprising a polyamide and the compound of formula (II), advantageously in an amount of from 0.05 to 10.0 wt%, preferably from 0.1 to 5.0 wt%, more preferably from 0.2 to 2.5 wt%, in particular from 0.25 to 1.0 wt%, based on the overall weight (100 wt%) of the polyamide composition

The polyamide composition may be in the form of a powder, a pellet, a fiber or any desired shaped article.

The present invention further provides a polyamide masterbatch comprising from 5 to 50 wt%, preferably from 10 to 40 wt%, more preferably from 15 to 30 wt%, in particular from 20 to 25 wt% of the compound of formula (II) and from 95 to 50 wt%, preferably from 90 to 60 wt%, more preferably from 85 to 70 wt%, in particular from 80 to 75 wt% of polyamide. The polyamide masterbatch is obtainable by mixing the individual constituents, preferably in powder or pellet form, and optionally by them melting together and subsequent pelletization.

The inventive compositions are obtainable via a process comprising the admixture of the compound of formula (II) or the aforementioned masterbatch with the above-described polymers in an appropriate processing equipment, for example in an extruder or kneader. The incorporation of the compound of formula (II) or the aforementioned masterbatch may be used either before or during melt-processing. The resultant mixture may optionally undergo subsequent shaping processes.

Shaping processes include, for example, extrusion processes, pelletization, injection molding techniques, blow molding, calendering, spinning, and web melt-blowing.

Alternatively, the inventive compositions are obtainable via a process for stabilizing a polyamide, characterized by the following steps
(a) adding the compound of formula (II) to the one or more monomers used for the polymerization of the polyamide in an amount of 0,001 to 1 wt.%, preferably of 0.01 to 0.5 wt.%, more preferably of 0.05 to 0.3 wt.%; based on the total weight of the one or more monomers,
(b) polymerizing the one or more monomers to produce the polyamide.

The monomers are for example ε-caprolactam, ω-aminolauric acid, laurolactam, hexamethylenediamine, 11-aminoundecanoic acid, terephthalic acid, terephthalic chloride, isophthalic acid, isophthalic chloride, adipic acid, tetramethylenediamine, phenylenediamine.

The inventive composition may additionally comprise further customary stabilizing agents as listed below.
1. Antioxidants
   1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methyl-cyclohexyl)-4,6-dimethylphenol, 2,6-dioctadecyl-4-methylphenol, 2,4,6-tricyclohexylphenol, 2,6-di-tert-butyl-4-methoxymethylphenol, nonylphenols which are linear or branched in the side chains, for example 2,6-di-nonyl-4-methylphenol, 2,4-dimethyl-6-(1 '-methylundec-1 '-yl)-phenol, 2,4-dimethyl-6-(1 '-methylheptadec-1'-yl)-phenol, 2,4-dimethyl-6-(1'-methyltridec-1'-yl)-phenol and mixtures thereof.
   1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol.
   1.3. Hydroquinones and alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate, bis-(3,5-di-tert-butyl-4-hydroxyphenyl)-adipate.
   1.4. Tocopherols, for example α-tocopherol, β-tocopherol, γ-tocopherol, δ-tocopherol and mixtures thereof (vitamin E).
   1.5. Hydroxylated thio-diphenyl ethers, for example 2,2'-thio-bis-(6-tert-butyl-4-methylphenol), 2,2'-thio-bis-(4-octylphenol), 4,4'-thio-bis-(6-tert-butyl-3-methylphenol), 4,4'-thio-bis-(6-tert-butyl-2-methylphenol), 4,4'-thio-bis-(3,6-di-sec-amylphenol), 4,4'-bis-(2, 6-di-methyl-4-hydroxyphenyl)-disulfide.
   1.6. Alkylidenebisphenols, for example 2,2'-methylene-bis(6-tert-butyl-4-methylphenol), 2,2'-methylene-bis(6-tert-butyl-4-ethylphenol), 2,2'-methylenebis[4-methyl-6-(α-methylcyclohexyl)phenol], 2,2'-methylene-bis(4-methyl-6-cyclohexylphenol), 2,2'-methylene-bis(6-nonyl-4-methylphenol), 2,2'-methylene-bis(4,6-di-tert-butylphenol), 2,2'-ethylidene-bis(4,6-di-tert-butylphenol), 2,2'-ethylidene-bis(6-tert-butyl-4-isobutylphenol), 2,2'-methylene-bis[6-(α-methylbenzyl)-4-nonylphenol], 2,2'-methylene-bis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methylphenyl)-3-n-dodecylmercaptobutane, ethylene glycol bis[3,3-bis(3'-tert-butyl-4'-hydroxyphenyl)butyrate], bis(3-tert-butyl-4-hydroxy-5-methylphenyl)dicyclopentadiene, bis[2-(3'-tert-butyl-2'-hydroxy-5'-methylbenzyl)-6-tert-butyl-4-methylphenyl]terephthalate, 1,1-bis(3,5-dimethyl-2-hydroxyphenyl)butane, 2,2-bis(3,5-di-tert-butyl-4-hydroxyphenyl)propane, 2,2-bis(5-tert-butyl-4-hydroxy-2-methylphenyl)-4-n-dodecylmercaptobutane, 1,1,5,5-tetra(5-tert-butyl-4-hydroxy-2-methylphenyl)pentane.
   1.7. O-, N- and S-benzyl compounds, for example 3,5,3',5'-tetra-tert-butyl-4,4'-dihydroxydibenzyl ether, octadecyl-4-hydroxy-3,5-dimethylbenzylmercaptoacetate, tridecyl-4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl)dithioterephthalate, bis(3,5-di-tert-butyl-4-hydroxybenzyl)sulfide, isooctyl-3,5-di-tert-butyl-4-hydroxybenzylmercaptoacetate.
   1.8. Hydroxybenzylated malonates, for example dioctadecyl-2,2-bis(3,5-di-tert-butyl-2-hydroxybenzyl)malonate, di-octadecyl-2-(3-tert-butyl-4-hydroxy-5-methylbenzyl)malonate, didodecylmercaptoethyl-2,2-bis(3,5-di-tert-butyl-4-hydroxybenzyl)malonate, bis[4-(1,1,3,3-tetramethylbutyl)phenyl]-2,2-bis(3,5-di-tert-butyl-4-hydroxybenzyl)malonate.
   1.9. Aromatic hydroxybenzyl compounds, for example 1,3,5-tris(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene, 2,4,6-tris(3,5-di-tert-butyl-4-hydroxybenzyl)phenol.
   1.10. Triazine compounds, for example 2,4-bis(octylmercapto)-6-(3,5-di-tert-butyl-4-hydroxyanilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris(3,5-di-tert-butyl-4-hydroxyphenoxy)-1,2,3-triazine, 1,3,5-tris(3,5-di-tert-butyl-4-hydroxybenzyl)isocyanurate, 1,3,5-tris(4-tert-butyl-3-hydroxy-2,6-dimethylbenzyl)isocyanurate, 2,4,6-tris(3,5-di-tert-butyl-4-hydroxyphenylethyl)-1,3,5-triazine, 1,3,5-tris(3,5-di-tert-butyl-4-hydroxyphenylpropionyl)hexahydro-1,3,5-triazine, 1,3,5-tris(3,5-dicyclohexyl-4-hydroxybenzyl)isocyanurate.
   1.11. Benzylphosphonates, for example dimethyl-2,5-di-tert-butyl-4-hydroxybenzylphosphonate, diethyl-3,5-di-tert-butyl-4-hydroxybenzylphosphonate, dioctadecyl-3,5-di-tert-butyl-4-hydroxybenzylphosphonate, dioctadecyl-5-tert-butyl-4-hydroxy-3-methylbenzylphosphonate, the calcium salt of the monoethyl ester of 3,5-di-tert-butyl-4-hydroxybenzylphosphonic acid.
   1.12. Acylaminophenols, for example 4-hydroxylauranilide, 4-hydroxystearanilide, octyl N-(3,5-di-tert-butyl-4-hydroxyphenyl)carbamate.
   1.13. Esters of 3-(3,5-di-tert-butyl-4-hydroxyphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl) oxamide, 3-thiaundecanol, 3-thiapentadecanol, trimethylhexanediol, trimethylolpropane, 4-hydroxymethyl-1 -phospha-2,6,7 -trioxabicyclo[2.2.2]octane.
   1.14. Esters of 3-(5-tert-butyl-4-hydroxy-3-methylphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7 -trioxabicyclo[2.2.2]octane; 3,9-bis[2-{3-(3-tert-butyl-4-hydroxy-5-methylphenyl)propionyloxy}-1,1-dimethylethyl]-2,4,8,10-tetraoxaspiro[5.5]undecane.
   1.15. Esters of 3-(3,5-dicyclohexyl-4-hydroxyphenyl)propionic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide,3-thiaundecanol, 3-thiapentadecanol, trimethylhexanediol, trimethylolpropane, 4-hydroxymethyl-1-phospha-2,6,7-trioxabicyclo[2.2.2]octane.
   1.16. Esters of 3,5-di-tert-butyl-4-hydroxyphenyl acetic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phosphat-2,6,7-trioxabicyclo[2.2.2]octane.
   1.17. Amides of 3-(3,5-di-tert-butyl-4-hydroxyphenyl)propionic acid e.g. N,N'-bis(3,5-di-tertbutyl-4-hydroxyphenylpropionyl)hexamethylenediamide, N,N'-bis(3,5-di-tert-butyl-4-hydroxyphenylpropionyl)trimethylenediamide, N,N'-bis(3,5-di-tert-butyl-4-hydroxyphenylpropionyl)hydrazide,N,N'-bis[2-(3-[3,5-di-tert-butyl-4-hydroxyphenyl]propionyloxy)ethyl]oxamide (Naugard^{®} XL-1).
   1.18. Ascorbic acid (vitamin C)
   1.19. Aminic antioxidants, for example N,N'-di-isopropyl-p-phenylenediamine, N,N'-di-sec-butyl-p-phenylenediamine, N,N'-bis(1 ,4-dimethylpentyl)-p-phenylenediamine, N,N'-bis(1-ethyl-3-methylpentyl)-p-phenylenediamine, N,N'-bis(1-methylheptyl)-p-phenylenediamine, N,N'-dicyclohexyl-p-phenylenediamine, N,N'-diphenyl-p-phenylenediamine, N,N'-bis(2-naphthyl)-pphenylenediamine, N-isopropyl-N'-phenyl-p-phenylenediamine, N-(1,3-dimethylbutyl)-N'-phenyl-p-phenylenediamine, N-(1-methylheptyl)-N'-phenyl-p-phenylenediamine, N-cyclohexyl-N'-phenyl-p-phenylenediamine, 4-(p-toluenesulfamoyl)diphenylamine, N,N'-dimethyl-N,N'-disec-butyl-p-phenylenediamine, diphenylamine, N-allyldiphenylamine, 4 isopropoxydiphenylamine, N-phenyl-1-naphthylamine, N-(4-tert-octylphenyl)-1-naphthylamine, N-phenyl-2-naphthylamine, octylated diphenylamine, for example p,p'-di-tert-octyldiphenylamine, 4-n-butylaminophenol, 4-butyrylaminophenol, 4-nonanoylaminophenol, 4-dodecanoylaminophenol, 4-octadecanoylaminophenol, bis(4-methoxyphenyl)amine, 2,6-di-tert-butyl-4-dimethylaminomethylphenol, 2,4'-diaminodiphenylmethane, 4,4'-diaminodiphenylmethane, N,N,N',N'-tetramethyl- 4,4'-diaminodiphenylmethane, 1,2-bis[(2-methylphenyl)amino]ethane, 1,2-bis(phenylamino)propane, (o-tolyl)biguanide, bis[4-(1',3'-dimethylbutyl)phenyl]amine, tert-octylated Nphenyl-1-naphthylamine, a mixture of mono- and dialkylated tert-butyl/tert-octyldiphenylamines, a mixture of mono- and dialkylated nonyldiphenylamines, a mixture of mono- and dialkylated dodecyldiphenylamines, a mixture of mono- and dialkylated isopropyl/isohexyldiphenylamines, a mixture of mono- and dialkylated tert-butyldiphenylamines, 2,3-dihydro-3,3-dimethyl-4H-1,4-benzothiazine, phenothiazine, a mixture of mono- and dialkylated tertbutyl/tert-octylphenothiazines, a mixture of mono- and dialkylated tert-octylphenothiazines, N-allylphenothiazine, N,N,N',N'-tetraphenyl-1,4-diaminobut-2-ene, N,N-bis(2,2,6,6-tetramethylpiperid-4-yl-hexamethylenediamine, bis(2,2,6,6-tetramethylpiperid-4-yl)sebacate, 2,2,6,6-tetramethylpiperidin-4-one, 2,2,6,6-tetramethylpiperidin-4-ol.
2. UV absorbers and light stabilisers
   2.1. 2-(2'-Hydroxyphenyl)benzotriazoles, for example 2-(2'-hydroxy-5'-methylphenyl)benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-ditert-butyl-2'-hydroxyphenyl)-5-chlorobenzotriazole, 2-(3'-tert-butyl-2'-hydroxy-5'-methylphenyl)-5-chlorobenzotriazole, 2-(3'-sec-butyl-5'-tert-butyl-2'-hydroxyphenyl)benzotriazole, 2-(2'-hydroxy-4'-octyloxyphenyl)benzotriazole, 2-(3',5'-di-tert-amyl-2'-hydroxyphenyl)benzotriazole, 2-(3',5'-bis(α,α-dimethylbenzyl)-2'-hydroxyphenyl)benzotriazole, 2-(3'-tert-butyl-2'-hydroxy-5'(2-octyloxycarbonylethyl)phenyl)-5-chlorobenzotriazole, 2-(3'-tert-butyl-5'-[2-(2-ethylhexyloxy) carbonylethyl]-2'-hydroxyphenyl)-5-chlorobenzotriazole, 2-(3'-tert-butyl-2'-hydroxy-5'-(2-methoxycarbonylethyl)phenyl)-5-chlorobenzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole, 2-(3'-tert-butyl-2'-hydroxy-5'-(2-isooctyloxycarbonylethyl)phenyl)benzotriazole, 2,2'-methylene-bis-[4-(1,1,3,3-tetramethylbutyl)-6-benzotriazole-2-ylphenol]; the transesterification product of 2-[3'-tert-butyl-5' -(2-methoxycarbonylethyl)-2'-hydroxyphenyl]-2H-benzotriazole with polyethylene glycol 300; [R-CH2CH2'COO-CH2CH2+ ,where R = 3'-tert-butyl-4'-hydroxy-5'-2H-benzotri-2-azol-2-ylphenyl, 2-[2'-hydroxy-3'-(α,α-dimethylbenzyl)-5'-(1,1,3,3-tetramethylbutyl)phenyl]benzotriazole; 2-[2'-hydroxy-3'-(1,1,3,3 tetramethylbutyl)-5'-(α,α-dimethylbenzyl)phenyl]benzotriazole.
   2.2. 2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives.
   2.3. Esters of substituted and unsubstituted benzoic acids, for example 4-tert-butylphenylsalicylate, phenyl salicylate, octylphenyl salicylate, dibenzoyl resorcinol, bis(4-tert-butylbenzoyl)resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate,2-methyl-4,6-di-tert-butylphenyl 3,5-di-tert-butyl-4-hydroxybenzoate.
   2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate and N-(β-carbomethoxy-β-cyanovinyl)-2-methylindoline.
   2.5. Nickel compounds, for example nickel complexes of 2,2'-thiobis[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of the mono-alkyl esters, e.g. the methyl or ethyl ester, of 4-hydroxy-3,5-di-tert-butylbenzylphosphonic acid, nickel complexes of ketoximes, e.g. of 2-hydroxy-4-methylphenylundecylketoxime, nickel complexes of 1-phenyl-4-lauroyl-5-hydroxypyrazole, with or without additional ligands.
   2.6. Sterically hindered amines, for example bis(2,2,6,6-tetramethyl-4-piperidyl)sebacate, bis(2,2,6,6-tetramethyl-4-piperidyl)succinate, bis(1,2,2,6,6-pentamethyl-4-piperidyl)sebacate, bis(1-octyloxy-2,2,6,6-tetramethyl-4-piperidyl)sebacate, bis(1,2,2,6,6-pentamethyl-4-piperidyl) n-butyl-3,5-di-tert-butyl-4-hydroxybenzylmalonate, the condensate of 1-(2-hydroxyethyl)- 2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl)nitrilotriacetate, tetrakis(2,2,6,6-tetramethyl-4-piperidyl)-1,2,3,4-butanetetracarboxylate, 1,1'-(1,2-ethanediyl)-bis(3,3,5,5-tetramethylpiperazinone), 4-benzoyl-2,2,6,6-tetramethylpiperidine, 4-stearyloxy-2,2,6,6-tetramethylpiperidine, bis(1,2,2,6,6-pentamethylpiperidyl)-2-n-butyl-2-(2-hydroxy-3,5-di-tert-butylbenzyl)malonate, 3-n-octyl-7,7,9,9-tetramethyl-1,3,8-triazaspiro[4.5]decane-2,4-dione, bis(1-octyloxy-2,2,6,6-tetramethylpiperidyl)sebacate, bis(1-octyloxy-2,2,6,6-tetramethylpiperidyl)succinate, linear or cyclic condensates of N,N'-bis(2,2,6,6-tetramethyl-4-piperidyl)hexamethylenediamine and 4-morpholino-2,6-dichloro-1,3,5-triazine, the condensate of 2-chloro-4,6-bis(4-n-butylamino-2,2,6,6-tetramethylpiperidyl)-1,3,5-triazine and 1,2-bis(3-aminopropylamino)ethane, the condensate of 2-chloro-4,6-di-(4-n-butylamino-1,2,2,6,6-pentamethylpiperidyl)-1,3,5-triazine and 1,2-bis(3-aminopropylamino)ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidine-2,5-dione, 3-dodecyl-1-(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensate of N,N'-bis(2,2,6,6-tetramethyl-4-piperidyl)hexamethylenediamine and 4-cyclohexylamino-2,6-dichloro-1,3,5-triazine, a condensate of 1,2-bis(3-aminopropylamino)ethane and 2,4,6-trichloro-1,3,5-triazine as well as 4-butylamino-2,2,6,6-tetramethylpiperidine (CAS Reg. No. [136504-96-6]); a condensate of 1,6-hexanediamine and 2,4,6-trichloro-1,3,5-triazine as well as N,N-dibutylamine and 4-butylamino-2,2,6,6-tetramethylpiperidine (CAS Reg. No. [192265-64-7]); N-(2,2,6,6-tetramethyl-4-piperidyl)-n-dodecylsuccinimide, N-(1,2,2,6,6-pentamethyl-4-piperidyl)-n-dodecylsuccinimide, 2-undecyl-7,7,9,9-tetramethyl-1-oxa-3,5-diaza-4-oxo-spiro[4,5]decane, a reaction product of 7,7,9,9-tetramethyl-2-cycloundecyl-1-oxa-3,5-diaza-4-oxospiro-[4,5]decane and epichlorohydrin, 1,1-bis(1,2,2,6,6-pentamethyl-4-piperidyloxycarbonyl)-2-(4-methoxyphenyl)ethene, N,N'-bis-formyl-N,N'-bis(2,2,6,6-tetramethyl-4-piperidyl)hexamethylenediamine, a diester of 4-methoxymethylenemalonic acid with 1,2,2,6,6-pentamethyl-4-hydroxypiperidine, poly[methylpropyl-3-oxy-4-(2,2,6,6-tetramethyl-4-piperidyl)]siloxane, a reaction product of maleic acid anhydride-α-olefin copolymer with 2,2,6,6-tetramethyl-4-aminopiperidine or 1,2,2,6,6-pentamethyl-4-aminopiperidine, N,N'-bis(2,2,6,6-tetramethyl-4-piperidyl)isophthalamide.
   2.7. Oxamides, for example 4,4'-dioctyloxyoxanilide, 2,2'-diethoxyoxanilide, 2,2'-dioctyloxy-5,5'-di-tert-butyloxanilide, 2,2'-didodecyloxy-5,5'-di-tert-butyloxanilide, 2-ethoxy-2'-ethyloxanilide, N,N'-bis(3-dimethylaminopropyl)oxamide, 2-ethoxy-5-tert-butyl-2'-ethyloxanilide and its mixture with 2-ethoxy-2'-ethyl-5,4'-di-tert-butyloxanilide, mixtures of o- and p-methoxy-disubstituted oxanilides and mixtures of o- and p-ethoxy-disubstituted oxanilides.
   2.8. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethyl)-1,3,5-triazine, 2-[2-hydroxy-4-(2-hydroxy-3-octyloxypropyloxy) phenyl]-4,6-bis(2 ,4-dimethyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3 dodecyloxypropoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxypropoxy)phenyl]-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2 hydroxypropyloxy]phenyl}-4,6-bis(2,4-dimethylphenyl)-1,3,5-triazine.
3. Metal deactivators, for example N,N'-diphenyloxamide, N-salicylal-N'-salicyloyl hydrazine, N,N'-bis(salicyloyl)hydrazine, N,N'-bis(3,5-di-tert-butyl-4-hydroxyphenylpropionyl) hydrazine, 3-salicyloylamino-1,2,4-triazole, bis(benzylidene)oxalyl dihydrazide, oxanilide, isophthaloyl dihydrazide, sebacoyl bisphenylhydrazide, N,N'-diacetyladipoyl dihydrazide, N,N'-bis(salicyloyl)oxalyl-dihydrazide, N,N'-bis(salicyloyl)thiopropionyl dihydrazide.
4. Phosphites and phosphonites, for example triphenyl phosphite, diphenylalkyl phosphites, phenyldialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl)pentaerythritol diphosphite, bis(2,4-dicumylphenyl)pentaerythritol diphosphite, bis(2,6-di-tert-butyl-4-methylphenyl)pentaerythritol diphosphite, diisodecyloxypentaerythritol diphosphite, bis(2,4-di-tert-butyl-6-methylphenyl)pentaerythritol diphosphite, bis(2,4,6-tris(tert-butylphenyl))pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenz[d,g]-1,3,2-dioxaphosphocin, bis(2,4-di-tert-butyl-6-methylphenyl)methyl phosphite, bis(2,4-di-tert-butyl-6-methylphenyl)ethyl phosphite, 6-fluoro-2,4,8,10-tetra-tert-butyl-12H-methyl-dibenz[d,g]-1,3,2-dioxaphosphocin, 2,2',2"-nitrilo[triethyltris(3,3',5,5'-tetra-tert-butyl-1,1'-biphenyl-2,2'-diyl)phosphite], 2-ethylhexyl(3,3',5,5'-tetra-tert-butyl-1,1'-biphenyl-2,2'-diyl)phosphite, 5-butyl-5-ethyl-2-(2,4,6-tri-tert-butylphenoxy)-1,3,2-dioxaphosphirane.
5. Hydroxylamines, for example N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.
6. Nitrones, for example N-benzyl-α-phenylnitrone, N-ethyl-α-methylnitrone, N-octyl-α-heptylnitrone, N-lauryl-α-undecylnitrone, N-tetradecyl-α-tridecylnitrone, N-hexadecyl-α-pentadecylnitrone, N-octadecyl-α-heptadecylnitrone, N-hexadecyl-α-heptadecylnitrone, N-octadecyl-α-pentadecylnitrone, N-heptadecyl-α-heptadecylnitrone, N-octadecyl-α-hexadecylnitrone, nitrone derived from N,N-dialkylhydroxylamine derived from hydrogenated tallow amine.
7. Thiosynergists, for example dilauryl thiodipropionate, distearyl thiodipropionate or dioctadecyl disulphide.
8. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulphide, pentaerythritol tetrakis(β-dodecylmercapto)propionate.
9. Polyamide stabilisers, for example copper salts in combination with iodides and/or phosphorus compounds and salts of divalent manganese.
10. Basic co-stabilisers, for example melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids, for example calcium stearate, zinc stearate, magnesium behenate, magnesium stearate, sodium ricinoleate and potassium palmitate, antimony pyrocatecholate or zinc pyrocatecholate.
11. Conventional nucleating agents, for example inorganic substances, such as talcum, metal oxides, such as titanium dioxide or magnesium oxide, phosphates, carbonates or sulfates of, preferably, alkaline earth metals; organic compounds, such as mono- or polycarboxylic acids and the salts thereof, e.g. 4-tert-butylbenzoic acid, adipic acid, diphenylacetic acid, sodium succinate or sodium benzoate; polymeric compounds, such as ionic copolymers (ionomers). Especially preferred are 1,3:2,4-bis(3',4'-dimethylbenzylidene) sorbitol, 1,3:2,4-di(paramethyldibenzylidene)sorbitol, and 1,3:2,4-di(benzylidene)sorbitol.
12. Other additives, for example plasticisers, lubricants, rheology additives, catalysts, flow control agents, optical brighteners, flameproofing agents, antistatic agents and blowing agents.
13. Benzofuranones and indolinones, for example those disclosed in US-A-4,325,863; US-A-4,338,244; US-A-5,175,312; US-A-5,216,052; US-A-5,252,643; DE-A-4316611; DE-A-4316622; DE-A-4316876; EP-A-0589839 or EP-A-0591102 or 3-[4-(2-acetoxyethoxy)phenyl]-5,7-di-tert-butylbenzofuran-2-one, 5,7-di-tert-butyl-3-[4-(2-stearoyloxyethoxy)phenyl]benzofuran-2-one, 3,3'-bis[5,7-di-tert-butyl-3-(4-[2-hydroxyethoxy]phenyl)benzofuran-2-one], 5,7-di-tert-butyl-3-(4-ethoxyphenyl)benzofuran-2-one, 3-(4-acetoxy-3,5-dimethylphenyl)-5,7-di-tert-butylbenzofuran-2-one, 3-(3,5-dimethyl-4-pivaloyloxyphenyl)-5,7-di-tert-butylbenzofu ran-2-one, 3-(3,4-dimethylphenyl)-5,7-di-tert-butylbenzofuran-2-one, 3-(2,3-dimethylphenyl)-5,7-di-tert-butylbenzofuran-2-one.

The present invention is further illustrated by the following examples and claims. The examples show the preferred embodiments of the invention.

### Examples

Unless otherwise stated all values given in wt.% are directed to % by weight and all ratio given are based on weight ratio.

The following substances were used for the Examples described below. The inventive stabilizer was produced as described in example 1:

**Table 1: Substances**

| **Abbreviation** | **Product Name** | **Producer supplier** | **Chemical class** |
|---|---|---|---|
| **Polymers** | | | |
| PA 6 | PA 6 UBE 1022B, RV 3.36 @ 96% H2SO4, Conc. 1% | Ube | Polyamide 6 |

| **HALS-Stabilizers** | | | |
|---|---|---|---|
| S1 | Dibutyl-N,N'-bis(2,2,6,6-tetramethyl-4-piperidinyl)-1,3-benzenedicarboxamide | Experimental Clariant product | LMW-HALS* based on n-butyl-TAD and isophthalic acid |
| S2 | Dibutyl-N,N'-bis(2,2,6,6-tetramethyl-4-piperidinyl)-1,4-benzenedicarboxamide | Experimental Clariant product | LMW-HALS* based on n-butyl-TAD and terephthalic acid |
| NS | Nylostab^{®} S-EED | Clariant SE | LMW-HALS* |

| | | | |
|---|---|---|---|
| * LMW HALS = low molecular weight HALS ** oligomeric HMW HALS = oligomeric high molecular weight HALS | | | |

**Table 2: Methods for substance characterization and evaluation of substance performance**

| | Method |
|---|---|
| Relative viscosity | ηᵣₑₗ = η/ηₛ |
| | ratio of the viscosity of a solution (η) to the viscosity of the solvent used (ηₛ) |
| Discoloration ΔE | |
| calculated from CIEL*a*b* color values as difference between the displayed color after exposure and the original color of the polymer samples. | CIE 1964 / observer 10 degree / Illuminant D 65 (with Spectrophotometer) |
| Yellowness-index (YI) | ASTM E 313 - 20 |
| Xenon Weathering Exposure Test | ISO 4892-2 wet/dry |

### A.) Preparation of stabilizers (S)

### Example 1: Preparation of N1,N3-dibutyl-N1,N3-bis(2,2,6,6-tetramethyl-4-piperidyl)benzene-dicarboxamide stabilizers (S)

### Example 1(A) (Preparation of S1):

A mixture of 600 g of n-propanol or 2-propanol, 300 g of deionized water and 212 g (1.0 mol) of n-butyl-TAD was charged into a reactor under nitrogen atmosphere. Afterwards, 108 g (0.53 mol) of isophthaloyl dichloride was added continuously under stirring. Then, 92 g (1.15 mol) of a 50 wt-% aqueous NaOH solution was added under continuous stirring at ambient conditions. Subsequently, it was stirred for 6 hours under reflux. The aqueous phase was separated off. Then, the organic solvent was replaced with water by continuous distillation of propanol and addition of water. Then, an organic solvent was added. Afterwards, the water phase was separated off and the organic phase was dried until completion. The product was obtained as a colourless to yellow clear melt 253,0 g (~91%). Purity: 99,4 area-% via GC.

### Example 1(B) (Preparation of S2):

A mixture of 600 g of n-propanol or 2-propanol, 300 g of deionized water and 212 g (1.0 mol) of n-butyl-TAD was charged into a reactor under nitrogen atmosphere. Afterwards, 108 g (0.53 mol) of terephthaloyl dichloride was added continuously under stirring. Then, 92 g (1.15 mol) of a 50 wt-% aqueous NaOH solution was added under continuous stirring at ambient conditions. Subsequently, it was stirred for 6 hours under reflux. The aqueous phase was separated off. Then, the organic solvent was replaced with water by continuous distillation of propanol and addition of water. Then the precipitant was filtered and washed with deionized water. The precipitant was dried to completion. The product was obtained as a white to off-white powder 244,8 g (~88 %). Purity: 98,5 area-% via GC.

### B.) Application tests in polyamides

### B.2) Application tests in PA 6

### Example 2: Preparation of the stabilizer masterbatch in PA 6 pellets comprising the inventive or reference stabilizer in a masterbatch form by twin-screw extrusion

The following PA 6 compound pellets were prepared using PA 6 polyamide (PA 6 UBE 1022B) and either S1, S2 or NS:
4 kg of PA 6 granules were mixed with 1 kg of the reference or inventive stabilizer in a plastic bag. Afterwards, the mixture was extruded using a Leistritz ZSE 27 maxx twin-screw extruder coupled with water bath and pelletizer. Ref Table 3

The processing temperature for the reference NS was set up to 290°C to ensure good dispersion. The processing temperature for the inventive stabilizers S1 and S2 was set to 250°C. (melting point difference)

The pellets were dried again in the vacuum oven at 80 °C under N₂ gas for a minimum of 12 hours until the moisture content is less than 0,05%.

**Table 3: Masterbatch compositions (all amounts given in % by weight referring to the total amount of polyamide and stabilizer)**

| **Masterbatch** | **Composition** |
|---|---|
| MB1 | PA 6 + 20% NS |
| MB2 | PA 6 + 20% S1 |
| MB3 | PA 6 + 20% S2 |

### Example 3: Preparation of the PA 6 compound pellets comprising the inventive or reference stabilizer using masterbatches

The following PA 6 compound pellets were prepared using PA 6 polyamide (PA 6 UBE 1022B) and the masterbatch of either the S1, S2 or NS prepared in example 2:
1970 g of PA 6 granules were mixed with 30 g, or 1950 g of PA 6 granules were mixed with 50 g of the masterbatch of either the reference or inventive stabilizer in a plastic bag to yield the final concentrations of the stabilizers in the PA 6 compound shown in Table 4. Afterwards, the mixture was extruded at 250°C in a Collin single-screw extruder (25 x 25D) with water bath and pelletized.

The pellets were dried again in the vacuum oven at 80 °C under N₂ gas for a minimum of 12 hours until the moisture content is less than 0,05%.

**Table 4: Stabilizer/polyamide compositions (all amounts given in % by weight referring to the total amount of polyamide and stabilizer)**

| **Formulation** | **Composition** |
|---|---|
| F1@240°C | PA 6 |
| F2@240°C | PA 6 + 0.3% NS |
| F3@240°C | PA 6 + 0.5% NS |
| F4@240°C | PA 6 + 0.3% S1 |
| F5@240°C | PA 6 + 0.5% S1 |
| F6@280°C | PA 6 + 0.3% S2 |
| F7@280°C | PA 6 + 0.5% S2 |

### Example 4: Evaluation of Yellowness Index of PA 6 plates after Xenon Weathering Exposure Test (WOM)

The pellets from example 3 above were processed in an Arburg injection molding machine at 250°C to form the injected plates with the following dimensions of the test specimen: plate size 10x10 cm2; plate thickness: 1 mm.

Surface cracking resistance, plate appearance and yellowing, caused by the Accelerated Artificial Weathering Exposure Test (WOM) were determined for the samples listed in Tables 5 and 5a using the testing methods as described in Table 1 above. The test results are summarized in Tables 5a and 5b.

**Table 5a: Yellowness Index (YI) after WOM exposure**

| YI | WOM time | Initial | 500 h | 1000 h | 1500 h | 2000 h | 2500 h | 3000 h | 3500 h | 4000 h | 4500 h | 5000 h |
|---|---|---|---|---|---|---|---|---|---|---|---|---|
| No. | Formulations | | | | | | | | | | | |
| Ex. 1 | F-1 | 2,9 | 3,2 | 4,3 | Sample disintegrated | | | | | | | |
| Ex. 2 | F-2 | 2,8 | 5,6 | 6,1 | 6,1 | 5,6 | 5,8 | 4,8 | 4,9 | 5,3 | Sample disintegrated | |
| Ex. 3 | F-3 | 2,0 | 5,9 | 6,5 | 6,8 | 6,6 | 6,9 | 6,1 | 6,2 | 6,8 | Sample disintegrated | |
| Ex. 4 | F-4 | 2,7 | 4,6 | 4,8 | 4,8 | 4,5 | 4,8 | 4,0 | 4,1 | 4,7 | 4,2 | Sample disintegrated |
| Ex. 5 | F-5 | 1,8 | 4,8 | 5,5 | 5,5 | 5,4 | 5,6 | 4,6 | 4,7 | 5,1 | 4,5 | Sample disintegrated |
| Ex. 6 | F-6 | 1,4 | 2,0 | 2,4 | 2,6 | 2,6 | 3,0 | 2,4 | 2,7 | 3,2 | 3,0 | Sample disintegrated |
| Ex. 7 | F-7 | 1,5 | 2,4 | 3,0 | 3,3 | 3,4 | 3,7 | 3,1 | 3,4 | 4,0 | 3,6 | Sample disintegrated |

**Table 5b: ΔE after WOM exposure**

| Delta E | WOM time | 500 h | 1000 h | 1500 h | 2000 h | 2500 h | 3000 h | 3500 h | 4000 h | 4500 h | 5000 h |
|---|---|---|---|---|---|---|---|---|---|---|---|
| No. | Formulations | | | | | | | | | | |
| Ex. 1 | F-1 | 0,2 | 1,4 | Sample disintegrated | | | | | | | |
| Ex. 2 | F-2 | 1,7 | 1,9 | 2,0 | 1,7 | 1,8 | 1,2 | 1,3 | 1,6 | Sample disintegrated | |
| Ex. 3 | F-3 | 2,2 | 2,6 | 2,8 | 2,7 | 2,8 | 2,4 | 2,5 | 2,9 | Sample disintegrated | |
| Ex. 4 | F-4 | 1,1 | 1,2 | 1,3 | 1,1 | 1,2 | 0,7 | 0,9 | 1,3 | 1,1 | Sample disintegrated |
| Ex. 5 | F-5 | 1,7 | 2,1 | 2,2 | 2,1 | 2,3 | 1,7 | 1,7 | 2,1 | 1,8 | Sample disintegrated |
| Ex. 6 | F-6 | 0,4 | 0,7 | 0,8 | 0,8 | 1,0 | 0,6 | 0,8 | 1,2 | 1,2 | Sample disintegrated |
| Ex. 7 | F-7 | 0,5 | 0,9 | 1,1 | 1,1 | 1,3 | 0,9 | 1,2 | 1,5 | 1,4 | Sample disintegrated |

Polymer stabilization is crucial to avoid sample disintegration to weathering conditions due to polymer degradation. The test plate which was not stabilized (Ex. 1), disintegrated after 1000 hours of WOM exposure, so its lifetime is very limited. Plates stabilized with NS (Ex. 2 and Ex. 3) are durable, but yellow and discolor strongly over time and disintegrated after 4000 hours of WOM exposure. S1 (Ex. 4 and Ex. 5) and S2 (Ex. 6 and Ex. 7) stabilized samples are more durable and disintegrate after 4500 hours of WOM exposure. Further, samples stabilized with S1 and S2 do not yellow so strongly than NS before disintegrating. After 4500 hours S2 shows the lowest yellowing (Ex. 6 YI = 3,0) which is less than the samples stabilized with S1 (Ex. 4 YI = 4,2) and NS (Ex. 2 YI = disintegrated). Similar results can be seen for the color retention (ΔE) after WOM exposure. Samples stabilized with S1 and S2 have a better color retention than NS before disintegrating as shown in Table 5a. Overall, S2 stabilized samples show the best protection against accelerated artificial weathering exposure.

## Claims

1. Use of the compound of formula (II) 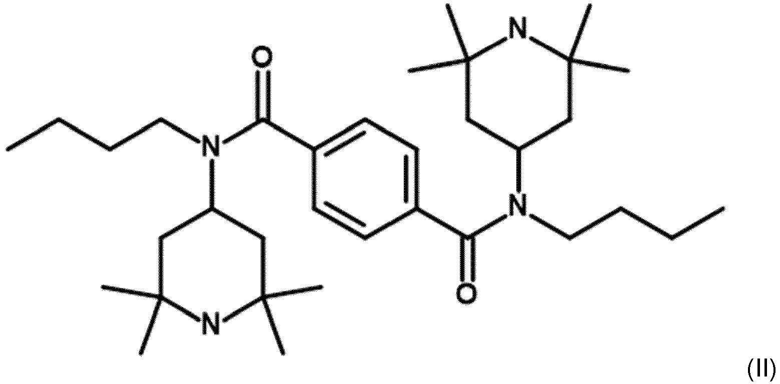 for enhancing the processing and long-term stability, such as the heat resistance, light resistance and oxidation resistance of polyamides.

2. Use according to claim 1, wherein the polyamides are PA 6 or PA 66 or mixtures thereof.

3. Polyamide composition, comprising a compound of formula (II) and a polyamide.

4. Polyamide composition according to claim 3, wherein the polyamide is PA 6 or PA 66 or mixtures thereof.

5. Polyamide composition according to claim 3 or 4, wherein the compound of formula (II) is present in the composition in an amount of 0.01 to 10.0 wt%, preferably of 0.05 to 5.0 wt%, more preferably of 0.08 to 2.5 wt%, particulary preferably from 0.1 to 1.0 wt%, based on the total weight (100 wt%) of the total composition.

6. Polyamide composition according to any one of claims 3 to 4, wherein the composition is a masterbatch.

7. Polyamide masterbatch according to claim 6, wherein the compound of formula (II) is present in the composition in an amount of 10 to 70 wt%, preferably of 20 to 60 wt%, more preferably of 20 to 40 wt%, based on the total weight (100 wt%) of the masterbatch.

8. Polyamide composition according to one or more of claims 3 to 7, wherein the composition comprises further stabilizing agents.

9. Process for stabilizing a polyamide, **characterized in that** the polyamide is mixed with the compound of formula (II) in an amount of 0,01 wt% to 8.0 wt%, preferably of 0.05 to 5.0 wt%, more preferably of 0.08 to 2.5 wt%, particularly preferably in an amount of 0.1 to 1.0 wt%; %, based on the total weight of the total composition, and
melt-processed.

10. Process for stabilizing a polyamide, **characterized in that** the polyamide is mixed with a masterbatch according to claim 7 in in an amount of 0.015 wt% - 90 wt%, preferably from 0.02 wt% to 60 wt% more preferably from 0.05wt% to 50 wt%, particularly preferably from 0.1 wt% to 40 wt%, based on the total weight of the total composition, and
melt-processed.

11. Process for stabilizing a polyamide, **characterized by** the following steps
(a) adding the compound of formula (II) to the one or more monomers used for the polymerization of the polyamide in an amount of 0,001 to 1 wt.%, preferably of 0.01 to 0.5 wt.%, more preferably of 0.05 to 0.3 wt.%; based on the total weight of the one or more monomers,
(b) polymerizing the one or more monomers to produce the polyamide.

12. Process according to claim 11, wherein the one or more monomers are selected from the group consisting of ε-caprolactam, ω-aminolauric acid, laurolactam, hexamethylenediamine, 11-aminoundecanoic acid, terephthalic acid, terephthalic chloride, isophthalic acid, isophthalic chloride, adipic acid, tetramethylenediamine, phenylenediamine, preferably selected from the group consisting of ε-caprolactam, hexamethylenediamine, adipic acid, more preferably selected from the group consisting of ε-caprolactam.
